(19) **Europäisches Patentamt / European Patent Office / Office européen des brevets**

(11) **EP 4 353 355 A1**

(12) **EUROPEAN PATENT APPLICATION**
published in accordance with Art. 153(4) EPC

(43) Date of publication:
**17.04.2024 Bulletin 2024/16**

(21) Application number: **22816114.7**

(22) Date of filing: **31.05.2022**

(51) International Patent Classification (IPC):
$B01J\ 23/04$ (2006.01)    $B01J\ 37/02$ (2006.01)
$B01J\ 37/04$ (2006.01)    $B01J\ 37/08$ (2006.01)
$C07B\ 61/00$ (2006.01)    $C07C\ 51/09$ (2006.01)
$C07C\ 57/04$ (2006.01)    $C07C\ 67/317$ (2006.01)
$C07C\ 69/653$ (2006.01)

(52) Cooperative Patent Classification (CPC):
**B01J 23/04; B01J 37/02; B01J 37/04; B01J 37/08;
C07B 61/00; C07C 51/09; C07C 57/04;
C07C 67/317; C07C 69/653**

(86) International application number:
**PCT/JP2022/022145**

(87) International publication number:
**WO 2022/255368 (08.12.2022 Gazette 2022/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **01.06.2021 JP 2021092244**

(71) Applicant: **Mitsubishi Chemical Corporation
Chiyoda-ku
Tokyo 100-8251 (JP)**

(72) Inventors:
• **HAYASHI, Akio
Tokyo 100-8251 (JP)**

• **TSUJIMOTO, Yuuki
Tokyo 100-8251 (JP)**
• **HASEGAWA, Toshio
Tokyo 100-8251 (JP)**
• **NISHIDA, Kazufumi
Tokyo 100-8251 (JP)**
• **FUJISUE, Masaya
Tokyo 100-8251 (JP)**
• **NINOMIYA, Wataru
Tokyo 100-8251 (JP)**

(74) Representative: **Hoffmann Eitle
Patent- und Rechtsanwälte PartmbB
Arabellastraße 30
81925 München (DE)**

(54) **CATALYST, METHOD FOR PRODUCING CATALYST, AND METHOD FOR PRODUCING UNSATURATED CARBOXYLIC ACID AND/OR UNSATURATED CARBOXYLIC ACID ESTER**

(57) An object of the present invention is to provide a catalyst that enables production of an unsaturated carboxylic acid and/or unsaturated carboxylic acid ester represented by methyl methacrylate with high selectivity. The object is achieved by a catalyst including: one or more elements selected from boron, magnesium, zirconium, hafnium, and titanium; one or more elements selected from alkali metal elements; and silica; the catalyst having a peak height ratio $I_2/I_1$ of 0 to 1.2, wherein $I_1$ represents the peak height at $417 \pm 10$ cm$^{-1}$, and $I_2$ represents the peak height at $1050 \pm 10$ cm$^{-1}$, as obtained by Raman spectroscopy.

Fig. 1

EP 4 353 355 A1

**Description**

TECHNICAL FIELD

The invention relates to a catalyst, a method for producing a catalyst, and a method for producing an unsaturated carboxylic acid and/or unsaturated carboxylic acid ester.

BACKGROUND ART

Methyl methacrylate is an extremely useful substance used as a raw material of various types of polymers for various applications. As methods for industrial production of methyl methacrylate, a number of methods such as the acetone cyanohydrin method and the direct oxidation method using a C4 material have been studied. In recent years, a production method called alpha method is attracting attention. In general, the alpha method includes: a first-stage reaction in which methyl propionate is produced using ethylene as a raw material; and a second-stage reaction in which the resulting methyl propionate is subjected to aldol condensation reaction, to produce methyl methacrylate. The method of producing methyl methacrylate by the alpha method particularly requires improvement of the selectivity of methyl methacrylate in the second-stage reaction, and a variety of catalysts have been studied therefor.

For example, as a catalyst for the second-stage reaction in the alpha method, Patent Document 1 proposes a catalyst comprising a porous, large-surface-area silica containing 1 to 10% by mass alkali metal, which catalyst contains a specific amount of a compound of at least one regulator element selected from boron, magnesium, aluminum, zirconium, and hafnium.

Patent Document 2 proposes a catalyst obtained by allowing at least one modifying metal selected from zirconium or hafnium as one or two metal atoms to be supported on a carrier, and subjecting the resulting product to calcination.

Patent Document 3 proposes a catalyst obtained by allowing at least one modifying metal selected from from boron, magnesium, aluminum, zirconium, hafnium, and titanium as one or two metal atoms to be supported on a carrier, and further allowing a catalytic metal element to be supported thereon, followed by subjecting the resulting product to calcination.

PRIOR ART DOCUMENTS

[Patent Documents]

[Patent Document 1] Japanese Translated PCT Patent Application Laid-Open No. 2002-511336
[Patent Document 2] WO 2019/53438
[Patent Document 3] WO 2020/183193

SUMMARY OF THE INVENTION

PROBLEMS TO BE SOLVED BY THE INVENTION

However, since the use of the catalysts described in Patent Documents 1 to 3 does not lead to sufficient selectivity of methyl methacrylate, industrial production of methyl methacrylate with higher selectivity has been demanded.

Thus, an object of the present invention is to provide a catalyst that enables highly selective production of an unsaturated carboxylic acid ester represented by methyl methacrylate and/or an unsaturated carboxylic acid.

MEANS FOR SOLVING THE PROBLEMS

The present invention was made in view of the above circumstances. The present inventors discovered that the above problem can be solved by using a catalyst whose peak intensity ratio as obtained by Raman spectroscopy is a specific value, thereby achieving the present invention.

More specifically, the present invention can be summarized as follows.

[1]: A catalyst comprising:

element X that is one or more selected from the group consisting of boron, magnesium, aluminum, zirconium, hafnium, and titanium;

element Y that is one or more selected from alkali metal elements; and
silica;

the catalyst having a peak height ratio $I_2/I_1$ of from 0 to 1.2, wherein $I_1$ represents the maximum peak height at $417\pm10$ cm$^{-1}$, and $I_2$ represents the maximum peak height at $1050\pm10$ cm$^{-1}$, as obtained by Raman spectroscopy.
[2]: The catalyst according to [1], wherein the $I_2/I_1$ is 0 to 0.5.
[3]: The catalyst according to [1] or [2], which is a catalyst for producing an unsaturated carboxylic acid and/or unsaturated carboxylic acid ester.
[4]: The catalyst according to any one of [1] to [3], wherein the element X comprises zirconium.
[5]: The catalyst according to any one of [1] to [4], wherein a content of the element X is from 0.3 to 10% by mass with respect to the total mass of the catalyst.
[6]: The catalyst according to any one of [1] to [5], wherein the element Y comprises cesium.
[7]: The catalyst according to any one of [1] to [6], wherein a content of the element Y is from 3 to 25% by mass with respect to the total mass of the catalyst.
[8]: The catalyst according to any one of [1] to [7], having a molar ratio MY/MX of from 1.3 to 8, wherein MX represents the number of moles of the element X, and MY represents the number of moles of the element Y.
[9]: The catalyst according to any one of [1] to [8], having a BET specific surface area of from 50 to 600 m$^2$/g.
[10]: A method of producing a catalyst, the method comprising steps of:

(1) impregnating a silica-containing carrier with a solution or dispersion (liquid A) containing element X that is one or more selected from the group consisting of boron, magnesium, aluminum, zirconium, hafnium, and titanium, to obtain a solid component A;
(2) subjecting the solid component A to heat treatment, to obtain a solid component B;
(3) impregnating the solid component B with a solution or dispersion (liquid B) containing element Y that is one or more selected from alkali metal elements, to obtain a solid component C; and
(4-1) subjecting the solid component C to heat treatment at a heat treatment temperature of from 100 to 300°C, to obtain a catalyst;

wherein in the Step (2), the solid component A is subjected to heat treatment at a heat treatment temperature of from 150°C to 300°C for from 15 minutes to 24 hours.
[11]: The method of producing a catalyst according to [10], wherein, in the Step (2), the heating rate from the time when the solid component A reaches 120°C to the time when the solid component A reaches the heat treatment temperature is not more than 10°C/minute.
[12]: The method of producing a catalyst according to [10] or [11], wherein, in the Step (4-1), the solid component C is subjected to heat treatment for from 15 minutes to 24 hours.
[13] : A method of producing a catalyst, the method comprising steps of:

(1) impregnating a silica-containing carrier with a solution or dispersion (liquid A) containing element X that is one or more selected from the group consisting of boron, magnesium, aluminum, zirconium, hafnium, and titanium, to obtain a solid component A;
(2) subjecting the solid component A to heat treatment, to obtain a solid component B;
(3) impregnating the solid component B with a solution or dispersion (liquid B) containing element Y that is one or more selected from alkali metal elements, to obtain a solid component C; and
(4-2) subjecting the solid component C to heat treatment at a heat treatment temperature of from 400 to 450°C, to obtain a catalyst;

wherein, in the Step (4-2), the heating rate from the time when the solid component C reaches 120°C to the time when the solid component C reaches the heat treatment temperature is not more than 10°C/minute.
[14]: The method of producing a catalyst according to [13], wherein, in the Step (4-2), the solid component C is subjected to heat treatment for from 3 to 24 hours.
[15]: The method of producing a catalyst according to [13] or [14], wherein, in the Step (4-2), the heating rate from the time when the solid component C reaches 120°C to the time when the solid component C reaches the heat treatment temperature is from 2 to 8°C/minute.
[16] : A method of producing a catalyst, the method comprising steps of:

(1) impregnating a silica-containing carrier with a solution or dispersion (liquid A) containing element X that is one or more selected from the group consisting of boron, magnesium, aluminum, zirconium, hafnium, and titanium, to obtain a solid component A;

(2) subjecting the solid component A to heat treatment, to obtain a solid component B;

(3) impregnating the solid component B with a solution or dispersion (liquid B) containing element Y that is one or more selected from alkali metal elements, to obtain a solid component C; and

(4-3) subjecting the solid component C to heat treatment at a heat treatment temperature of more than 450°C and not more than 700°C, to obtain a catalyst; wherein, in the Step (4-3), the heating rate from the time when the solid component C reaches 120°C to the time when the solid component C reaches the heat treatment temperature is not more than 10°C/minute.

[17]: The method of producing a catalyst according to [16], wherein, in the Step (4-3), the solid component C is subjected to heat treatment for from 15 minutes to 24 hours.

[18]: The method of producing a catalyst according to [16] or [17], wherein, in the Step (4-3), the heating rate from the time when the solid component C reaches 120°C to the time when the solid component C reaches the heat treatment temperature is from 2 to 8°C/minute.

[19]: The method of producing a catalyst according to any one of [13] to [18], wherein, in the Step (2), the solid component A is subjected to heat treatment at a heat treatment temperature of from 100 to 200°C.

[20]: The method of producing a catalyst according to any one of [13] to [19], wherein, in the Step (2), the solid component A is subjected to heat treatment for from 15 minutes to 24 hours.

[21]: The method of producing a catalyst according to any one of [10] to [20], wherein the catalyst is a catalyst for producing an unsaturated carboxylic acid and/or unsaturated carboxylic acid ester.

[22]: The method of producing a catalyst according to any one of [10] to [21], wherein, in the Step (1), the element X comprises zirconium.

[23]: The method of producing a catalyst according to any one of [10] to [22], wherein, in the Step (3), the element Y comprises cesium.

[24] : A method of producing an unsaturated carboxylic acid and/or unsaturated carboxylic acid ester, the method comprising using the catalyst according to any one of [1] to [9], to produce an unsaturated carboxylic acid and/or unsaturated carboxylic acid ester from a carboxylic acid and/or carboxylic acid ester, and formaldehyde.

[25]: A method of producing an unsaturated carboxylic acid and/or unsaturated carboxylic acid ester, the method comprising using a catalyst produced by the method of producing a catalyst according to any one of [10] to [23], to produce an unsaturated carboxylic acid and/or unsaturated carboxylic acid ester from a carboxylic acid and/or carboxylic acid ester, and formaldehyde.

EFFECT OF THE INVENTION

The present invention can provide a catalyst that enables highly selective production of a desired product, in particular, an unsaturated carboxylic acid and/or unsaturated carboxylic acid ester, and a method of producing the catalyst. The present invention can also provide a method of producing an unsaturated carboxylic acid and/or unsaturated carboxylic acid ester using the catalyst with a high selectivity.

BRIEF DESCRIPTION OF THE DRAWINGS

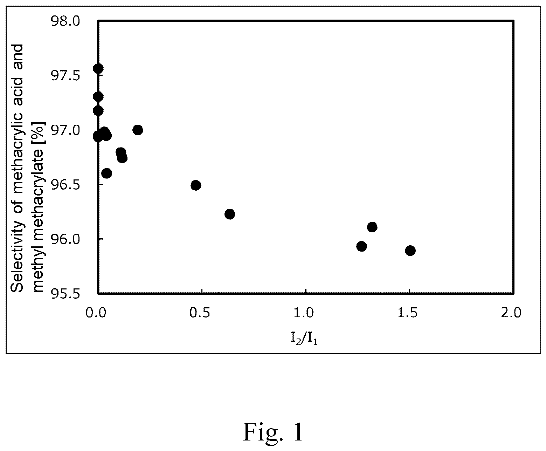

Fig. 1 is a graph illustrating the relationship between the peak height ratio $I_2/I_1$, as obtained by Raman spectroscopy, of a catalyst according to an embodiment of the present invention, and the selectivity of methacrylic acid and methyl methacrylate in the production of the methacrylic acid and methyl methacrylate using the catalyst.

MODE FOR CARRYING OUT THE INVENTION

Embodiments of the present invention are described below, but the present invention is not limited to the following.

In the present description, a numerical range expressed using "to" means the range including the values described before and after "to" as the lower limit value and the upper limit value, respectively. Thus, "A to B" means not less than A and not more than B.

[Catalyst]

One embodiment of the present invention is a catalyst comprising:

element X that is one or more selected from the group consisting of boron, magnesium, aluminum, zirconium, hafnium, and titanium;

element Y that is one or more selected from alkali metal elements; and
silica;

the catalyst having a peak height ratio $I_2/I_1$ of from 0 to 1.2, wherein $I_1$ represents the maximum peak height at $417\pm10$ cm$^{-1}$, and $I_2$ represents the maximum peak height at $1050\pm10$ cm$^{-1}$, as obtained by Raman spectroscopy.

The shape of the catalyst is not limited, and examples of the shape include a spherical shape, columnar shape, and ring shape.

<Raman Spectroscopy of Catalyst>

In Raman spectroscopy of the catalyst, the peak at $417\pm10$ cm$^{-1}$ is assumed to be derived from the structure of the active sites of the main reaction, and the peak at $1050\pm10$ cm$^{-1}$ is assumed to be derived from the structure of the active sites of the side reaction.

In cases where the peak height ratio $I_2/I_1$ is from 0 to 1.2, the catalyst is thought to be in a state where the active sites of the side reaction are decreased, and where the active sites of the main reaction are appropriately highly dispersed, so that the selectivity of the desired product is thought to be improved.

From the viewpoint of improving the selectivity of the desired product, the value of $I_2/I_1$ is preferably not more than 1, more preferably not more than 0.5, still more preferably not more than 0.3 in terms of the upper limit.

A catalyst whose $I_2/I_1$ is within this range can be obtained, for example, by adjusting the heat treatment temperature in the production of the catalyst. In particular, it is preferred to use a method in which a solid component A and a solid component C are subjected to heat treatment under the conditions described in the method of producing a catalyst described below.

In the present invention, the Raman spectroscopy is performed using a laser Raman spectrometer at an excitation wavelength of 488 nm. The laser Raman spectrometer used may be, for example, a 3D laser Raman spectrometer Nanofinder (manufactured by Tokyo Instruments, Inc.). The $I_2/I_1$ according to Raman spectroscopy is measured by the following procedure.

The catalyst is divided into two portions, and Raman spectroscopy is carried out at five points on the resulting cross-section while changing the measurement site from one end to another end at regular intervals. Subsequently, the Raman spectrum obtained is corrected using, as a baseline, the line connecting the minimum intensity at $730\pm2$ cm$^{-1}$ and the minimum intensity at $867\pm2$ cm$^{-1}$. In the Raman spectrum after the baseline correction, the length of the perpendicular line from the peak top of the peak at $417\pm10$ cm$^{-1}$ to the baseline is defined as $I_1$. The length of the perpendicular line from the peak top of the peak at $1050\pm10$ cm$^{-1}$ to the line drawn between both ends of the peak is defined as $I_2$. The above operation is carried out for the five points where the analysis was carried out, and $I_1$, $I_2$, and $I_2/I_1$ are calculated as arithmetic averages.

<Constituent Elements of Catalyst>

The catalyst of the present embodiment contains element X that is one or more selected from the group consisting of boron, magnesium, aluminum, zirconium, hafnium, and titanium. The element X preferably contains boron or zirconium, more preferably contains zirconium. The element X may be either one kind of element or two or more kinds of elements.

From the viewpoint of improving the selectivity of the desired product, the content of the element X is preferably not less than 0.3% by mass, more preferably not less than 0.5% by mass, still more preferably not less than 1% by mass, especially preferably not less than 1.5% by mass in terms of the lower limit with respect to the total mass of the catalyst. The content is preferably not more than 10% by mass, more preferably not more than 6% by mass, still more preferably not more than 5% by mass, especially preferably not more than 4% by mass in terms of the upper limit.

The catalyst of the present embodiment contains element Y that is one or more selected from alkali metal elements. The element Y preferably contains lithium, sodium, potassium, rubidium, or cesium, more preferably contains potassium, rubidium, or cesium, still more preferably contains cesium. The element Y may be either one kind of element or two or more kinds of elements.

From the viewpoint of improving the selectivity of the desired product, the content of the element Y is preferably not less than 3% by mass, more preferably not less than 4% by mass, still more preferably not less than 6% by mass in terms of the lower limit with respect to the total mass of the catalyst. The content is preferably not more than 25% by mass, more preferably not more than 18% by mass, still more preferably not more than 14% by mass, especially preferably not more than 12% by mass in terms of the upper limit.

From the viewpoint of improving the selectivity of the desired product, when the number of moles of the element X is defined as MX, and the number of moles of the element Y is defined as MY, the molar ratio MY/MX is preferably not less than 1.3, more preferably not less than 1.5, still more preferably not less than 1.7, especially preferably not less than 1.9 in terms of the lower limit. The ratio is preferably not more than 8, more preferably not more than 6, still more preferably not more than 5.5, especially preferably not more than 5 in terms of the upper limit.

The contents of the element X and the element Y can be measured by X-ray fluorescence analysis. From the contents of the element X and the element Y obtained, the molar ratio MY/MX can be calculated. The X-ray fluorescence analysis is carried out, for example, using ZSX Primus IV (manufactured by Rigaku Corporation) in a helium atmosphere.

The catalyst of the present embodiment contains silica. In the catalyst, the silica functions as a carrier. In addition to silica, the carrier may contain alumina, zeolite, titania, zirconia, or the like.

The catalyst of the present embodiment may also contain a metal element other than those described above. Examples of the other metal element include iron. From the viewpoint of suppressing a decrease in the selectivity of the desired product, the content of the other metal element is preferably not more than 1% by mass, more preferably not more than 0.5% by mass, still more preferably not more than 0.2% by mass in terms of the upper limit with respect to the total mass of the catalyst.

The catalyst of the present embodiment may also contain another element derived from the production process of the catalyst.

<BET Specific Surface Area of Catalyst>

From the viewpoint of improving the selectivity of the desired product, the BET specific surface area of the catalyst is preferably not less than 50 $m^2$/g, more preferably not less than 90 $m^2$/g, still more preferably not less than 100 $m^2$/g in terms of the lower limit. The BET specific surface area is preferably not more than 600 $m^2$/g, more preferably not more than 500 $m^2$/g, still more preferably not more than 350 $m^2$/g, especially preferably not more than 300 $m^2$/g in terms of the upper limit.

The measurement of the BET specific surface area is carried out by calculation by the BET one-point method using a nitrogen adsorption measurement apparatus. Examples of the nitrogen adsorption measurement apparatus that may be employed include Macsorb (manufactured by Mountech Co., Ltd.).

[Method of Producing Catalyst 1]

A first embodiment of the method of producing a catalyst of the present invention includes the following steps.

(1) A step of impregnating a silica-containing carrier with a solution or dispersion (liquid A) containing element X that is one or more selected from the group consisting of boron, magnesium, aluminum, zirconium, hafnium, and titanium, to obtain a solid component A.
(2) A step of subjecting the solid component A to heat treatment, to obtain a solid component B.
(3) A step of impregnating the solid component B with a solution or dispersion (liquid B) containing element Y that is one or more selected from alkali metal elements, to obtain a solid component C.
(4-1) A step of subjecting the solid component C to heat treatment at a heat treatment temperature of from 100 to 300°C, to obtain a catalyst.

In the first embodiment of the method of producing a catalyst of the present invention, in the Step (2), the solid component A is subjected to heat treatment at a heat treatment temperature of from 150°C to 300°C for from 15 minutes to 24 hours.

Each step is described below in detail.

<Step (1)>

In Step (1), a silica-containing carrier is impregnated with a solution or dispersion (liquid A) containing element X that is one or more selected from the group consisting of boron, magnesium, aluminum, zirconium, hafnium, and titanium, to obtain solid component A.

(Liquid A)

Liquid A can be obtained by dissolving or dispersing a compound containing element X in a solvent. In this process, the compound containing element X may be dissolved or dispersed while stirring the solvent.

The element X preferably contains boron or zirconium, more preferably contains zirconium. The element X may be either one kind of element or two or more kinds of elements.

The compound containing element X is preferably an inorganic salt of the element X. The compound containing element X is preferably an inorganic salt of the element X. The inorganic salt is not limited as long as it is an inorganic compound containing no hydrocarbon. Examples of the inorganic salt include carbonates, nitrates, oxynitrates, sulfates, acetates, ammonium salts, oxides, and halides. These may be used individually or in combination. More specifically, in cases where the element X is boron, examples of the inorganic salt include boron oxide. In cases where the element X is magnesium, examples of the inorganic salt include magnesium nitrate, magnesium sulfate, magnesium carbonate, and magnesium acetate. In cases where the element X is zirconium, examples of the inorganic salt include zirconium oxynitrate, zirconium nitrate, zirconium sulfate, zirconium carbonate, zirconium perchlorate, and zirconium acetate. The inorganic salt preferably contains zirconium oxynitrate. In cases where the element X is hafnium, examples of the inorganic salt include hafnium nitrate, hafnium sulfate, hafnium perchlorate, and hafnium acetate. In cases where the element X is titanium, examples of the inorganic salt include titanium oxide, titanium chloride, and titanyl sulfate.

From the viewpoint of improving the selectivity of the desired product, the content of the element X in the resulting catalyst is preferably not less than 0.3% by mass, more preferably not less than 0.5% by mass, still more preferably not less than 1% by mass, especially preferably not less than 1.5% by mass in terms of the lower limit with respect to the total mass of the catalyst. The content is preferably not more than 10% by mass, more preferably not more than 6% by mass, still more preferably not more than 5% by mass, especially preferably not more than 4% by mass in terms of the upper limit. The content of the element X can be adjusted based on the amount of the element X added.

The solvent is not limited, and examples of the solvent include water and an organic solvent. The organic solvent is preferably an alcohol, more preferably an alcohol having 1 to 6 carbon atoms, still more preferably an alcohol having 1 to 3 carbon atoms, especially preferably an alcohol having 1 to 2 carbon atoms, most preferably methanol.

The amount of the compound containing element X, contained in the liquid A, is not limited. From the viewpoint of uniformly dispersing the element X, the amount of the compound is preferably not less than 2 mmol, more preferably not less than 5 mmol, still more preferably not less than 10 mmol in terms of the lower limit with respect to 100 mL of the solvent. From the viewpoint of suppressing reaggregation of the element X, the amount of the compound is preferably not more than 60 mmol, more preferably not more than 50 mmol, still more preferably not more than 40 mmol in terms of the upper limit with respect to 100 mL of the solvent.

(Carrier)

The carrier to be impregnated with liquid A contains silica, and may also contain alumina, zeolite, titania, zirconia, or the like. As the silica, silica gel is preferably used.

From the viewpoint of improving the selectivity of the desired product, the BET specific surface area of the carrier is preferably not less than 50 $m^2/g$, more preferably not less than 90 $m^2/g$, still more preferably not less than 100 $m^2/g$ in terms of the lower limit. The BET specific surface area of the carrier is preferably not more than 600 $m^2/g$, more preferably not more than 500 $m^2/g$, still more preferably not more than 350 $m^2/g$, especially preferably not more than 300 $m^2/g$ in terms of the upper limit.

The method of adjusting the BET specific surface area of the carrier is not limited. For example, a carrier having pores with which a desired BET specific surface area can be obtained may be used. In cases where the proportion of pores formed in the carrier is large, the BET specific surface area tends to be large. In cases where the proportion of pores formed in the carrier is small, the BET specific surface area tends to be small.

The measurement of the BET specific surface area is carried out by calculation by the BET one-point method using a nitrogen adsorption measurement apparatus. Examples of the nitrogen adsorption measurement apparatus that may be employed include Macsorb (manufactured by Mountech Co., Ltd.).

The shape of the carrier is not limited, and examples of the shape include a powder shape, granular shape, pellet shape, and tablet shape.

The average particle size of the carrier is not limited. From the viewpoint of suppressing the pressure loss in the reaction to produce the desired product, the average particle size is preferably not less than 500 $\mu$m, more preferably not less than 1 mm, still more preferably not less than 1.3 mm in terms of the lower limit. From the viewpoint of suppressing the progression of the side reaction, the average particle size is preferably not more than 10 mm, more preferably not more than 6 mm, still more preferably not more than 5 mm in terms of the upper limit.

The average pore size of the carrier is not limited. From the viewpoint of suppressing the progression of the side reaction, the average pore size is preferably not less than 3 nm, more preferably not less than 5 nm, still more preferably not less than 7 nm in terms of the lower limit. From the viewpoint of maintaining the BET specific surface area, the average pore size is preferably not more than 200 nm, more preferably not more than 150 nm, still more preferably not more than 100 nm, especially preferably not more than 50 nm in terms of the upper limit.

The carrier is preferably subjected to calcination before impregnated with liquid A, to remove water. The carrier after the calcination is preferably stored in a desiccator, dry air, or dry inert gas so as to maintain the water-free state.

(Impregnation of Carrier with Liquid A)

The method of impregnating the carrier with liquid A is not limited, and a known method may be used therefor. Examples of the method include the pore filling method, in which liquid A in the same amount as the pore volume of the carrier is added to the carrier, and the immersion method, in which the carrier is immersed in liquid A.

The length of time for which the carrier is impregnated with the liquid A is not limited. From the viewpoint of sufficiently impregnating the carrier with the element X, the length of time is preferably not less than 15 minutes, more preferably not less than 1 hour in terms of the lower limit. From the viewpoint of the length of time required for producing the catalyst, the length of time for which the carrier is impregnated with the liquid A is preferably not more than 50 hours, more preferably not more than 30 hours, still more preferably not more than 24 hours in terms of the upper limit.

The amount of the liquid A with respect to the carrier is not limited. From the viewpoint of uniformly impregnating the carrier with the element X, the amount of the liquid A is preferably not less than 0.9 times the pore volume of the carrier in terms of the lower limit. From the viewpoint of reducing the amount of the solvent used, the amount of the liquid A is preferably not more than 10 times, more preferably not more than 5 times, still more preferably not more than twice the pore volume of the carrier in terms of the upper limit.

By impregnating the carrier with liquid A, a solid component A is obtained. The solid component A is preferably obtained by removal of the solvent. The solvent can be removed by a known method such as a method using a rotary evaporator, or a method by filtration separation.

<Step (2)>

In the Step (2), the solid component A obtained in the Step (1) is subjected to heat treatment, to obtain a solid component B.

The heat treatment temperature is from 150 and 300°C. The heat treatment temperature is preferably not less than 170°C, more preferably not less than 190°C in terms of the lower limit. It is thought that an active-site structure suitable for producing the desired product is formed by performing heat treatment at from 150 to 300°C in the Step (2), and also performing heat treatment under the conditions in the Step (4-1) described later.

The heating rate from the time when the solid component A reaches 120°C to the time when the solid component A reaches the heat treatment temperature is preferably not more than 10°C/minute, more preferably not more than 5°C/minute. While removal of the solvent of the solid component A mainly occurs before the temperature reaches 120°C, a change in the active-site structure of the solid component A occurs at not less than 120°C. It is thought that an active-site structure suitable for producing the desired product is formed by setting the heating rate at not less than 120°C to the condition described above.

The heat treatment time is ranging from 15 minutes to 24 hours. The heat treatment time is preferably not less than 30 minutes, more preferably not less than 1 hour, still more preferably not less than 5 hours, especially preferably not less than 10 hours in terms of the lower limit. The heat treatment time is preferably not more than 20 hours, more preferably not more than 15 hours in terms of the upper limit.

<Step (3)>

In the Step (3), the solid component B obtained in the Step (2) is impregnated with a solution or dispersion (liquid B) containing element Y that is one or more selected from alkali metal elements, to obtain a solid component C.

(Liquid B)

Liquid B can be obtained by dissolving or dispersing a compound containing element Y in a solvent. In this process, the compound containing element Y may be dissolved or dispersed while stirring the solvent.

The element Y preferably contains lithium, sodium, potassium, rubidium, or cesium, more preferably contains potassium, rubidium, or cesium, still more preferably contains cesium. The element Y may be either one kind of element or two or more kinds of elements.

The compound containing element Y is preferably a salt of the element Y. The salt is not limited, and examples of the salt include carbonates, nitrates, sulfates, acetates, ammonium salts, oxides, and halides. These may be used individually or in combination. More specifically, in cases where the element Y is lithium, examples of the salt include lithium carbonate and lithium nitrate. In cases where the element Y is sodium, examples of the salt include sodium carbonate, sodium nitrate, and sodium sulfate. In cases where the element Y is potassium, examples of the salt include potassium carbonate, potassium nitrate, and potassium sulfate. In cases where the element Y is rubidium, examples of the salt include rubidium carbonate, rubidium nitrate, and rubidium sulfate. In cases where the element Y is cesium, examples of the salt include cesium carbonate, cesium bicarbonate, cesium nitrate, and cesium sulfate.

From the viewpoint of improving the selectivity of the desired product, the content of the element Y in the resulting catalyst is preferably not less than 3% by mass, more preferably not less than 4% by mass, still more preferably not less than 6% by mass in terms of the lower limit with respect to the total mass of the catalyst. The content is preferably not more than 25% by mass, more preferably not more than 18% by mass, still more preferably not more than 14% by mass, especially preferably not more than 12% by mass in terms of the upper limit. The content of the element X can be adjusted based on the amount of the element X added.

From the viewpoint of improving the selectivity of the desired product, when the number of moles of the element X is defined as MX, and the number of moles of the element Y is defined as MY, the molar ratio MY/MX in the resulting catalyst is preferably not less than 1.3, more preferably not less than 1.5, still more preferably not less than 1.7, especially preferably not less than 1.9 in terms of the lower limit. The ratio is preferably not more than 8, more preferably not more than 6, still more preferably not more than 5.5, especially preferably not more than 5 in terms of the upper limit. MY/MX can be adjusted based on the amount of the element Y added with respect to the element X.

As the solvent, the same solvent as in the liquid A may be used.

The amount of the compound containing element Y, contained in the liquid B, is not limited. From the viewpoint of improving the selectivity of the desired product, the amount of the compound is preferably not less than 6 mmol, more preferably not less than 14 mmol, still more preferably not less than 25 mmol in terms of the lower limit with respect to 100 mL of the solvent. The amount of the compound is preferably not more than 60 mmol, more preferably not more than 50 mmol, still more preferably not more than 40 mmol in terms of the upper limit with respect to 100 mL of the solvent.

The resulting liquid B is preferably left to stand before the solid component B is impregnated therewith. By this, the average particle size of the compound containing element Y in the liquid B tends to decrease, and the element Y tends to be appropriately highly dispersed in the resulting catalyst, leading to improved selectivity of the desired product. The standing time of the liquid B is preferably not less than 15 minutes in terms of the lower limit, and not more than 50 hours in terms of the upper limit.

(Impregnation of Solid Component B with Liquid B)

The method of impregnating the solid component B with the liquid B is not limited, and a known method may be used therefor. Examples of the method include the pore filling method, in which liquid B in the same amount as the pore volume of the solid component B is added to the solid component B, and the immersion method, in which the solid component B is immersed in the liquid B.

The length of time for which the solid component B is impregnated with the liquid B is not limited. From the viewpoint of sufficiently impregnating the solid component B with the element Y, the length of time is preferably not less than 15 minutes, more preferably not less than 1 hour in terms of the lower limit. From the viewpoint of the length of time required for producing the catalyst, the length of time for which the solid component B is impregnated with the liquid B is preferably not more than 50 hours, more preferably not more than 30 hours, still more preferably not more than 24 hours in terms of the upper limit.

The amount of the liquid B with respect to the solid component B is not limited. From the viewpoint of uniformly impregnating the solid component B with the element Y, the amount of the liquid B is preferably not less than 0.9 times the pore volume of the solid component B in terms of the lower limit. From the viewpoint of reducing the amount of the solvent used, the amount of the liquid B is preferably not more than 10 times, more preferably not more than 5 times the pore volume of the solid component B in terms of the upper limit.

By impregnating the solid component B with the liquid B, a solid component C is obtained. The solid component C is preferably obtained by removal of the solvent. The solvent can be removed by a known method such as a method using a rotary evaporator, or a method by filtration separation.

<Step (4-1)>

In the Step (4-1), the solid component C obtained in the Step (3) is subjected to heat treatment at a heat treatment temperature of from 100 to 300°C, to obtain a catalyst. By the heat treatment, the solvent remaining in the solid component C, and unnecessary elements, that is, elements other than the element X, the element Y, and silica, can be removed.

Further, by carrying out the heat treatment under such conditions, a catalyst having a peak height ratio $I_2/I_1$ within the predetermined range as obtained by Raman spectrometry, and showing high selectivity of the desired product, can be obtained. This is thought to be because, by carrying out the heat treatment under the predetermined conditions in the Step (2), and by carrying out the heat treatment in the Step (4-1), an active-site structure suitable for producing the desired product can be formed in the resulting catalyst. In other words, the active sites of the side reaction decrease, and the active-site component that causes the main reaction is appropriately highly dispersed.

From the viewpoint of removal of the solvent, the heat treatment temperature is preferably not less than 110°C, more preferably not less than 120°C in terms of the lower limit. Further, from the viewpoint of improving the selectivity of the desired product, the heat treatment temperature is preferably not more than 250°C in terms of the upper limit.

The heat treatment time is preferably not less than 15 minutes, more preferably not less than 30 minutes, still more preferably not less than 1 hour, especially preferably not less than 5 hours, most preferably not less than 10 hours in terms of the lower limit. The heat treatment time is preferably not more than 24 hours, more preferably not more than 20 hours, still more preferably not more than 15 hours in terms of the upper limit.

The catalyst can be produced in such a manner.

[Method of Producing Catalyst 2]

A second embodiment of the method of producing a catalyst of the present invention includes the following steps.

(1) A step of impregnating a silica-containing carrier with a solution or dispersion (liquid A) containing element X that is one or more selected from the group consisting of boron, magnesium, aluminum, zirconium, hafnium, and titanium, to obtain a solid component A.
(2) A step of subjecting the solid component A to heat treatment, to obtain a solid component B.
(3) A step of impregnating the solid component B with a solution or dispersion (liquid B) containing element Y that is one or more selected from alkali metal elements, to obtain a solid component C.
(4-2) A step of subjecting the solid component C to heat treatment at a heat treatment temperature of from 400 to 450°C, to obtain a catalyst.

In the second embodiment of the method of producing a catalyst of the present invention, in the Step (4-2), the heating rate from the time when the solid component C reaches 120°C to the time when the solid component C reaches the heat treatment temperature is not more than 10°C/minute.

Each step is described below in detail.

<Step (1)>

The Step (1), and preferred embodiments thereof are the same as those in the first embodiment.

<Step (2)>

In the Step (2), the solid component A obtained in the Step (1) is subjected to heat treatment, to obtain a solid component B.

The heat treatment temperature is preferably from 100 to 200°C. By this, an active-site structure more suitable for producing the desired product is thought to be formed by performing the heat treatment under the conditions in the Step (4-2) described later. The heat treatment temperature is more preferably not less than 110°C in terms of the lower limit. The heat treatment temperature is more preferably not more than 150°C, still more preferably not more than 130°C in terms of the upper limit.

The heat treatment time is preferably not less than 15 minutes, more preferably not less than 1 hour, still more preferably not less than 5 hours, especially preferably not less than 10 hours in terms of the lower limit. The heat treatment time is preferably not more than 24 hours, more preferably not more than 20 hours, still more preferably not more than 15 hours in terms of the upper limit.

<Step (3)>

The Step (3), and preferred embodiments thereof are the same as those in the first embodiment.

<Step (4-2)>

In the Step (4-2), the solid component C obtained in the Step (3) is subjected to heat treatment at a heat treatment temperature of from 400 to 450°C, to obtain a catalyst. By the heat treatment, the solvent remaining in the solid component C, and unnecessary elements, that is, elements other than the element X, the element Y, and silica, can be removed.

Further, by carrying out the heat treatment under such conditions, a catalyst having a peak height ratio $I_2/I_1$ within the predetermined range as obtained by Raman spectrometry, and showing high selectivity of the desired product, can be obtained. This is thought to be because, by carrying out the heat treatment in the Step (4-2), an active-site structure suitable for producing the desired product can be formed in the resulting catalyst. In other words, the active sites of the side reaction decrease, and the active-site component that causes the main reaction is appropriately highly dispersed.

The heating rate from the time when the solid component C reaches 120°C to the time when the solid component C reaches the heat treatment temperature is not more than 10°C/minute. While removal of the solvent of the solid component C mainly occurs before the temperature reaches 120°C, a change in the active-site structure of the solid component C occurs at not less than 120°C. It is thought that an active-site structure suitable for producing the desired product is formed by setting the heating rate at not less than 120°C to the condition described above. The heating rate from the time when the solid component C reaches 120°C to the time when the solid component C reaches the heat treatment temperature is more preferably not less than 2°C/minute in terms of the lower limit, and not more than 8°C/minute in terms of the upper limit.

From the viewpoint of reducing the active sites of the side reaction in the solid component C, the heat treatment time is preferably not less than 3 hours, more preferably not less than 4 hours, still more preferably not less than 6 hours in terms of the lower limit. The heat treatment time is preferably not more than 24 hours, more preferably not more than 20 hours, still more preferably not more than 15 hours in terms of the upper limit.

The catalyst can be produced in such a manner.

[Method of Producing Catalyst 3]

A third embodiment of the method of producing a catalyst of the present invention includes the following steps.

(1) A step of impregnating a silica-containing carrier with a solution or dispersion (liquid A) containing element X that is one or more selected from the group consisting of boron, magnesium, aluminum, zirconium, hafnium, and titanium, to obtain a solid component A.
(2) A step of subjecting the solid component A to heat treatment, to obtain a solid component B.
(3) A step of impregnating the solid component B with a solution or dispersion (liquid B) containing element Y that is one or more selected from alkali metal elements, to obtain a solid component C.
(4-3) A step of subjecting the solid component C to heat treatment at a heat treatment temperature of more than 450°C and not more than 700°C, to obtain a catalyst.

In the third embodiment of the method of producing a catalyst of the present invention, in the Step (4-3), the heating rate from the time when the solid component C reaches 120°C to the time when the solid component C reaches the heat treatment temperature is not more than 10°C/minute.

Each step is described below in detail.

<Step (1)>

The Step (1), and preferred embodiments thereof are the same as those in the first embodiment.

<Step (2)>

The Step (2), and preferred embodiments thereof are the same as those in the second embodiment.

<Step (3)>

The Step (3), and preferred embodiments thereof are the same as those in the first embodiment.

<Step (4-3)>

In the Step (4-3), the solid component C obtained in the Step (3) is subjected to heat treatment at a heat treatment temperature of more than 450°C and not more than 700°C, to obtain a catalyst. By the heat treatment, the solvent remaining in the solid component C, and unnecessary elements, that is, elements other than the element X, the element Y, and silica, can be removed.

Further, by carrying out the heat treatment under such conditions, a catalyst having a peak height ratio

$I_2/I_1$ within the predetermined range as obtained by Raman spectrometry, and showing high selectivity of the desired product, can be obtained. This is thought to be because, by carrying out the heat treatment in Step (4-3), an active-site structure suitable for producing the desired product can be formed in the resulting catalyst. In other words, the active sites of the side reaction decrease, and the active-site component that causes the main reaction is appropriately highly dispersed.

From the viewpoint of improving the selectivity of the desired product, the heat treatment temperature is preferably not less than 500°C, more preferably not less than 550°C in terms of the lower limit. The heat treatment temperature is preferably not more than 650°C, more preferably not more than 600°C in terms of the upper limit.

The heating rate from the time when the solid component C reaches 120°C to the time when the solid component C reaches the heat treatment temperature is not more than 10°C/minute. While removal of the solvent of the solid component C mainly occurs before the temperature reaches 120°C, a change in the active-site structure of the solid component C occurs at not less than 120°C. It is thought that an active-site structure suitable for producing the desired product is formed by setting the heating rate at not less than 120°C to the condition described above. The heating rate from the time when the solid component C reaches 120°C to the time when the solid component C reaches the heat treatment temperature is more preferably not less than 2°C/minute in terms of the lower limit, and not more than 8°C/minute in terms of the upper limit.

From the viewpoint of reducing the active sites of the side reaction in the solid component C, the heat treatment time is preferably not less than 15 minutes, more preferably not less than 1 hour, still more preferably not less than 3 hours, especially preferably not less than 10 hours, most preferably not less than 15 hours in terms of the lower limit. The heat treatment time is preferably not more than 48 hours, more preferably not more than 24 hours in terms of the upper limit.

The catalyst can be produced in such a manner.

[Method of Producing Unsaturated Carboxylic Acid and/or Unsaturated Carboxylic Acid Ester]

By reacting a carboxylic acid and/or carboxylic acid ester with formaldehyde in the presence of a catalyst according to one embodiment of the present invention or a catalyst produced by a production method according to one embodiment of the present invention, a corresponding unsaturated carboxylic acid and/or unsaturated carboxylic acid ester can be produced. Thus, another embodiment of the present invention is a method of producing an unsaturated carboxylic acid and/or unsaturated carboxylic acid ester using the above catalyst from a carboxylic acid and/or carboxylic acid ester, and formaldehyde. By this, an unsaturated carboxylic acid and/or unsaturated carboxylic acid ester can be produced with a high selectivity.

The carboxylic acid and/or carboxylic acid ester is/are preferably a compound(s) represented by the following Formula (I).

$$R^1\text{-}CH_2\text{-}COOR^2 \; ... \; \text{Formula} \qquad (I)$$

In Formula (I), $R^1$ and $R^2$ each independently represent a hydrogen atom or an alkyl group having 1 to 12 carbon atoms. The compound represented by Formula (I) is preferably methyl propionate. The catalyst according to the present embodiment is especially effective in a method in which methacrylic acid and/or methyl methacrylate is/are produced from methyl propionate and formaldehyde.

In the reaction of the carboxylic acid and/or carboxylic acid ester with formaldehyde, a reaction raw material containing the carboxylic acid and/or carboxylic acid ester and formaldehyde is brought into contact with the catalyst, to produce the unsaturated carboxylic acid and/or unsaturated carboxylic acid ester.

In the reaction raw material, the molar ratio of formaldehyde to the total number of moles of the carboxylic acid and carboxylic acid ester is not limited, and is preferably from 0.05 to 20 from the viewpoint of improving the selectivity of the desired product. The molar ratio is more preferably not less than 0.2 in terms of the lower limit, and not more than 15 in terms of the upper limit.

From the viewpoint of improving the selectivity of the desired product, the reaction is preferably carried out in the presence of an alcohol. When the reaction is carried out in the presence of the alcohol, the molar ratio of the alcohol to the total number of moles of the carboxylic acid and/or carboxylic acid ester is not limited, and is preferably 0.05 to 20. The molar ratio is more preferably not less than 0.1 in terms of the lower limit, and not more than 10 in terms of the upper limit.

Further, as long as the effect of the present invention is not remarkably impaired, compounds other than the those described above may be included. Examples of the other compounds include water.

The reaction temperature in the above reaction is not limited. From the viewpoint of improving the selectivity of the desired product, the reaction temperature is preferably not less than 100°C, more preferably not less than 200°C, still more preferably not less than 250°C in terms of the lower limit. The reaction temperature is preferably not more than 400°C, more preferably not more than 370°C, still more preferably not more than 360°C in terms of the upper limit.

The contacting time between the reaction raw material and the catalyst in the reaction is not limited. From the viewpoint of improving the selectivity of the desired product, the contacting time is preferably not less than 0.1 seconds, more preferably not less than 1 second, still more preferably not less than 2 seconds in terms of the lower limit. From the viewpoint of suppressing the progression of the side reaction, the contacting time is preferably not more than 100 seconds, more preferably not more than 50 seconds, still more preferably not more than 30 seconds.

The carboxylic acid and/or carboxylic acid ester contained in the reaction raw material can be produced by a known method. In cases where methyl propionate is used as the carboxylic acid and/or carboxylic acid ester, it can be produced by the carbonylation reaction of ethylene. The carbonylation reaction of ethylene is a method in which ethylene and carbon monoxide are reacted in the presence of a catalyst that enables the carbonylation of ethylene, to produce methyl propionate. The carbonylation reaction of ethylene is described below in detail.

The amount of the ethylene with respect to the carbon monoxide is not limited. The amount of the ethylene is preferably not less than 0.01 mol, more preferably not less than 0.1 mol in terms of the lower limit with respect to 100 mol of the carbon monoxide. The amount of the ethylene is preferably not more than 100 mol, more preferably not more than 10 mol in terms of the upper limit.

The reaction temperature in the carbonylation reaction is not limited. The reaction temperature is preferably not less than 20°C, more preferably not less than 40°C, still more preferably not less than 70°C in terms of the lower limit. The reaction temperature is preferably not more than 250°C, more preferably not more than 150°C, still more preferably not more than 120°C in terms of the upper limit. The reaction time is not limited, and is preferably from 0.1 to 100 hours.

The catalyst that enables the carbonylation of the ethylene is not limited, and a known catalyst may be used therefor. Examples of the catalyst include palladium catalysts containing a phosphine-based ligand. Specific examples of a palladium catalyst that may be used include the palladium catalyst described in Japanese Translated PCT Patent Application Laid-Open No. 10-511034. The palladium catalyst can be produced by a known method.

The carbonylation reaction is preferably carried out in the presence of an alcohol. The alcohol is not limited, and examples of the alcohol include methanol, ethanol, propanol, 2-propanol, 2-butanol, and t-butyl alcohol. Among these, methanol or ethanol is preferred. A single kind of alcohol may be used alone, or two or more kinds of alcohols may be used in combination.

The amount of the ethylene with respect to the alcohol is not limited. The amount of the ethylene is preferably not less than 0.01 mol, more preferably not less than 0.1 mol in terms of the lower limit with respect to 100 mol of the alcohol. The amount of the ethylene is preferably not more than 100 mol, more preferably not more than 10 mol in terms of the upper limit.

In the carbonylation reaction, the carbon monoxide may be fed together with an inert gas. Examples of the inert gases include hydrogen, nitrogen, carbon dioxide, and argon.

By the above method, an unsaturated carboxylic acid and/or unsaturated carboxylic acid ester can be produced, while the produced unsaturated carboxylic acid and/or unsaturated carboxylic acid ester usually contains an impurity. Thus, to remove the impurity, the unsaturated carboxylic acid and/or unsaturated carboxylic acid ester obtained is/are preferably purified by a known method such as distillation. The conditions of the purification may be appropriately adjusted such that an unsaturated carboxylic acid and/or unsaturated carboxylic acid ester with a desired purity can be obtained.

EXAMPLES

The present invention is described in detail by way of Examples and Comparative Examples. However, the present invention is not limited to the following Examples.

(Heat Treatment)

In Examples, the heat treatments of the solid component A and the solid C component were carried out using a muffle furnace. As the muffle furnace, Product Number FUM142PA (manufactured by ADVANTEC Co., Ltd.) or Product Number DF62 (manufactured by Yamato Scientific Co., Ltd.) was used.

(Raman Spectrometry)

The result of Raman spectrometry of the catalyst was obtained using a 3D laser Raman spectrometer Nanofinder (manufactured by Tokyo Instruments, Inc.) at an excitation wavelength of 488 nm. The $I_2/I_1$ was measured by the following procedure.

The catalyst was divided into two portions, and Raman spectroscopy was carried out at five points on the resulting cross-section while changing the measurement site from one end to another end at regular intervals. The Raman spectrum obtained was corrected using, as a baseline, the line connecting the minimum intensity at $730 \pm 2$ cm$^{-1}$ and the minimum intensity at $867 \pm 2$ cm$^{-1}$. In the Raman spectrum after the baseline correction, the length of the perpendicular line from the peak top of the peak at $417 \pm 10$ cm$^{-1}$ to the baseline was defined as $I_1$.

The length of the perpendicular line from the peak top of the peak at $1050 \pm 10$ cm$^{-1}$ to the line drawn between both ends of the peak was defined as $I_2$. In cases where no peak was present near $1050 \pm 10$ cm$^{-1}$, $I_2$ was regarded as

0. The above operation was carried out for the five points where the analysis was carried out, and $I_1$, $I_2$, and $I_2/I_1$ were calculated as arithmetic averages.

(X-Ray Fluorescence Analysis)

The contents of the element X and the element Y were measured by X-ray fluorescence analysis. From the contents of the element X and the element Y obtained, the molar ratio between the element X and the element Y, MY/MX, was calculated. The X-ray fluorescence analysis was carried out using ZSX Primus IV (manufactured by Rigaku Corporation) in a helium atmosphere.

(Measurement of BET Specific Surface Area)

The BET specific surface area of the catalyst was calculated by the BET one-point method using a nitrogen adsorption measurement apparatus Macsorb (manufactured by Mountech Co., Ltd.).

(Reaction Evaluation)

Reaction evaluation of the catalyst was carried out using as an example the reaction in which methacrylic acid and/or methyl methacrylate is/are produced from methyl propionate and formaldehyde. The reaction raw material and the reaction product in the reaction evaluation were analyzed using gas chromatography (Shimadzu Corporation, GC-2010). From the analysis result, the selectivity of methacrylic acid and methyl methacrylate was calculated by the following Formula (II).

```
Selectivity of methacrylic acid and methyl

methacrylate (%) = (the number of moles of the

methacrylic acid and methyl methacrylate produced)/(the

number of moles of the methyl propionate reacted) ×

100 ... Formula (II)
```

<Example 1>

After dissolving 4.5 g of zirconium oxynitrate dihydrate (Kishida Chemical Co., Ltd., special grade) in 135 mL of methanol (Nacalai Tesque, Inc., special grade), the resulting solution was left to stand for 24 hours, to prepare liquid A. As a carrier, 60 g of CARiACT Q-15 (Fuji Silysia Chemical Ltd.; particle size, 1.7 to 4 mm; average pore size, 10 nm) was used. The carrier was immersed in liquid A, and left to stand for 3.5 hours. Subsequently, the solvent was removed using a rotary evaporator, to obtain a solid component A.
The solid component A obtained was subjected to heat treatment at a heat treatment temperature of 120°C for 14 hours, to obtain a solid component B. In this process, the heating rate of the solid component A was 0.17°C/minute.
Thirty grams of the solid component B obtained was immersed for 3.5 hours in liquid B prepared by dissolving 4.9 g of cesium carbonate (Wako Pure Chemical Industries, Ltd., first grade) in 65 mL of methanol. Subsequently, the solution was removed by filtration, to obtain a solid component C.
The solid component C obtained was subjected to heat treatment at a heat treatment temperature of 500°C for 3 hours, to obtain a catalyst. In this process, the heating rate of the solid component C was 5°C/minute. The results of Raman spectrometry, X-ray fluorescence analysis, and measurement of the BET specific surface area of the catalyst are shown in Table 1.
Subsequently, reaction evaluation was carried out by the following procedure. About 3 g of the obtained catalyst was filled into a reactor. Subsequently, a reaction raw material liquid containing methyl propionate, methanol, formaldehyde, and water at a molar ratio of 1:1.40:0.19:0.5 was allowed to flow in an evaporator at 300°C at a flow rate of 0.160 mL/minute under normal pressure, and then allowed to flow in a reactor at 330°C for 16 hours. Subsequently, a reaction raw material liquid containing methyl propionate, methanol, formaldehyde, and water at a molar ratio of 1:0.64:0.27:0.01 was allowed to flow in an evaporator at 300°C under normal pressure, and then fed into a reactor at 330°C, to produce methacrylic acid and methyl methacrylate. The vapor from the outlet of the reactor was cooled to condensate, and the reaction product was collected. The analysis result of the obtained reaction product is shown in Table 1. Fig. 1 shows the relationship between the peak height ratio $I_2/I_1$ obtained by Raman spectrometry and the selectivity of methacrylic acid and methyl methacrylate.

<Examples 2 to 8>

A solid component C was each obtained by the same method as in Example 1.
The solid component C obtained was each subjected to heat treatment in the same manner as in Example 1 except that the heat treatment temperature and the heat treatment time were different as shown in Table 1, to obtain a catalyst. The results of Raman spectrometry, X-ray fluorescence analysis, and measurement of the BET specific surface area of the catalyst are shown in Table 1.
Subsequently, reaction evaluation was carried out in the same manner as in Example 1. The analysis result of the reaction product is shown in Table 1. Fig. 1 shows the relationship between the peak height ratio $I_2/I_1$ obtained by Raman spectrometry and the selectivity of methacrylic acid and methyl methacrylate.

<Example 9>

A solid component A was obtained by the same method as in Example 1.
The solid component A obtained was subjected to heat treatment in the same manner as in Example 1 except that the heat treatment temperature and the heat treatment time were different as shown in Table 1, to obtain a solid component B.
Using the solid component B obtained, a solid component C was obtained by the same method as in Example 1.
The solid component C obtained was subjected to heat treatment at a heat treatment temperature of 120°C for 14 hours, to obtain a catalyst. In this process, the heating rate of the solid component C was 0.17°C/minute. The results of Raman spectrometry, X-ray fluorescence analysis, and measurement of the BET specific surface area of the catalyst are shown in Table 1.
Subsequently, reaction evaluation was carried out in the same manner as in Example 1. The analysis result of the reaction product is shown in Table 1. Fig. 1 shows the relationship between the peak height ratio $I_2/I_1$ obtained by Raman spectrometry and the selectivity of methacrylic acid and methyl methacrylate.

<Examples 10 and 11>

A solid component A was each obtained by the same method as in Example 1.
The solid component A obtained was each subjected to heat treatment in the same manner as in Example 1 except that the heat treatment temperature and the heat treatment time were different as shown in Table 1, to obtain a solid component B.
Using the solid component B obtained, a solid component C was each obtained by the same method as in Example 1.
The solid component C obtained was each subjected to heat treatment in the same manner as in Example 9, to obtain a catalyst. The results of Raman spectrometry, X-ray fluorescence analysis, and measurement of the BET specific surface area of the catalyst are shown in Table 1.
Subsequently, reaction evaluation was carried out in the same manner as in Example 1. The analysis result of the reaction product is shown in Table 1. Fig. 1 shows the relationship between the peak height ratio $I_2/I_1$ obtained by Raman spectrometry and the selectivity of methacrylic acid and methyl methacrylate.

<Example 12>

A solid component A was obtained by the same method as in Example 1.
The solid component A obtained was subjected to heat treatment at a heat treatment temperature of 200°C for 14 hours, to obtain a solid component B. In this process, the heating rate of the solid component A was 5°C/minute.
Using the solid component B obtained, a solid component C was obtained by the same method as in Example 1.
The solid component C obtained was subjected to heat treatment in the same manner as in Example 9, to obtain a catalyst. The results of Raman spectrometry, X-ray fluorescence analysis, and measurement of the BET specific surface area of the catalyst are shown in Table 1.
Subsequently, reaction evaluation was carried out in the same manner as in Example 1. The analysis result of the reaction product is shown in Table 1. Fig. 1 shows the relationship between the peak height ratio $I_2/I_1$ obtained by Raman spectrometry and the selectivity of methacrylic acid and methyl methacrylate.

<Example 13>

A solid component C was obtained by the same method as in Example 1.

The solid component C obtained was subjected to heat treatment in the same manner as in Example 1 except that different heat treatment temperature and heat treatment time were employed as shown in Table 1, to obtain a catalyst. The results of Raman spectrometry, X-ray fluorescence analysis, and measurement of the BET specific surface area of the catalyst are shown in Table 1.

Subsequently, reaction evaluation was carried out in the same manner as in Example 1. The analysis result of the reaction product is shown in Table 1. Fig. 1 shows the relationship between the peak height ratio $I_2/I_1$ obtained by Raman spectrometry and the selectivity of methacrylic acid and methyl methacrylate.

<Comparative Example 1>

A solid component C was obtained by the same method as in Example 1.

The solid component C obtained was subjected to heat treatment in the same manner as in Example 9, to obtain a catalyst. The results of Raman spectrometry, X-ray fluorescence analysis, and measurement of the BET specific surface area of the catalyst are shown in Table 1.

Subsequently, reaction evaluation was carried out in the same manner as in Example 1. The analysis result of the reaction product is shown in Table 1. Fig. 1 shows the relationship between the peak height ratio $I_2/I_1$ obtained by Raman spectrometry and the selectivity of methacrylic acid and methyl methacrylate.

<Comparative Example 2>

A solid component C was obtained by the same method as in Example 1.

The solid component C obtained was subjected to heat treatment in the same manner as in Example 1 except that different heat treatment temperature and heat treatment time were employed as shown in Table 1, to obtain a catalyst. The results of Raman spectrometry, X-ray fluorescence analysis, and measurement of the BET specific surface area of the catalyst are shown in Table 1.

Subsequently, reaction evaluation was carried out in the same manner as in Example 1. The analysis result of the reaction product is shown in Table 1. Fig. 1 shows the relationship between the peak height ratio $I_2/I_1$ obtained by Raman spectrometry and the selectivity of methacrylic acid and methyl methacrylate.

<Comparative Example 3>

A solid component C was obtained by the same method as in Example 1.

The solid component C obtained was subjected to heat treatment at a heat treatment temperature of 450°C for 3 hours, to obtain a catalyst. In this process, the heating rate of the solid component C was 25°C/minute. The results of Raman spectrometry, X-ray fluorescence analysis, and measurement of the BET specific surface area of the catalyst are shown in Table 1.

Subsequently, reaction evaluation was carried out in the same manner as in Example 1. The analysis result of the reaction product is shown in Table 1. Fig. 1 shows the relationship between the peak height ratio $I_2/I_1$ obtained by Raman spectrometry and the selectivity of methacrylic acid and methyl methacrylate.

Table 1

| | Heat treatment conditions of solid component A | | | Heat treatment conditions of solid component C | | | $I_2/I_1$ | Content with respect to the total mass of the catalyst | | MY/MX | BET specific surface area [m²/g] | Selectivity of methacrylic acid and methyl methacrylate [%] |
|---|---|---|---|---|---|---|---|---|---|---|---|---|
| | Heating time [° C/minute ] | Heat treatment temperature [°C] | Heat treatment time [hours] | Heating time [° C/minute ] | Heat treatment temperature [°C] | Heat treatment time [hours] | | Zr [% by mass] | Cs [% by mass] | | | |
| Example 1 | 0.17 | 120 | 14 | 5 | 500 | 3 | 0.47 | 2.2 | 8.7 | 2.7 | 176 | 96.49 |
| Example 2 | 0.17 | 120 | 14 | 5 | 500 | 6 | 0.04 | 2.2 | 8.7 | 2.7 | 166 | 96.95 |
| Example 3 | 0.17 | 120 | 14 | 5 | 500 | 10 | 0.03 | 2.2 | 8.7 | 2.7 | 166 | 96.98 |
| Example 4 | 0.17 | 120 | 14 | 5 | 500 | 24 | 0.00 | 2.2 | 8.7 | 2.7 | 162 | 97.31 |
| Example 5 | 0.17 | 120 | 14 | 5 | 600 | 3 | 0.00 | 2.2 | 8.7 | 2.7 | 141 | 96.94 |
| Example 6 | 0.17 | 120 | 14 | 5 | 600 | 6 | 0.00 | 2.2 | 8.7 | 2.7 | 166 | 96.95 |
| Example 7 | 0.17 | 120 | 14 | 5 | 600 | 10 | 0.00 | 2.2 | 8.7 | 2.7 | 146 | 97.18 |
| Example 8 | 0.17 | 120 | 14 | 5 | 600 | 24 | 0.00 | 2.2 | 8.7 | 2.7 | 133 | 97.56 |
| Example 9 | 0.17 | 200 | 14 | 0.17 | 120 | 14 | 0.11 | 2.1 | 8.3 | 2.7 | 147 | 96.79 |
| Example 10 | 0.17 | 250 | 14 | 0.17 | 120 | 14 | 0.12 | 2.2 | 8.8 | 2.7 | 149 | 96.74 |
| Example 11 | 0.17 | 300 | 14 | 0.17 | 120 | 14 | 0.04 | 2.2 | 8.6 | 2.7 | 146 | 96.60 |
| Example 12 | 5 | 200 | 14 | 0.17 | 120 | 14 | 0.19 | 2.3 | 8.8 | 2.6 | 157 | 97.00 |
| Example 13 | 0.17 | 120 | 14 | 5 | 450 | 3 | 0.63 | 2.2 | 8.7 | 2.7 | 179 | 96.23 |
| Comperative Example 1 | 0.17 | 120 | 14 | 0.17 | 120 | 14 | 1.50 | 2.2 | 8.7 | 2.7 | 183 | 95.89 |
| Comperative Example 2 | 0.17 | 120 | 14 | 5 | 380 | 3 | 1.27 | 2.2 | 8.7 | 2.7 | 185 | 95.93 |
| Comperative Example 3 | 0.17 | 120 | 14 | 25 | 450 | 3 | 1.32 | 2.6 | 9.0 | 2.4 | 157 | 96.11 |

EP 4 353 355 A1

17

As can be seen in Table 1 and Fig. 1, Examples 1 to 13, which used catalysts having peak height ratios $I_2/I_1$ within the predetermined range, showed largely improved selectivities of methacrylic acid and methyl methacrylate as compared to Comparative Examples 1 to 3, which used catalysts having peak height ratios $I_2/I_1$ outside the predetermined range.

It can also be seen that Examples 1 to 13, which used catalysts prepared with heat treatment conditions for solid components A and solid components C within the predetermined ranges, showed largely improved selectivities of methacrylic acid and methyl methacrylate as compared to Comparative Examples 1 to 3, which used catalysts prepared with heat treatment conditions for solid components A and solid components C outside the predetermined ranges.

## Claims

1. A catalyst comprising:

    element X that is one or more selected from the group consisting of boron, magnesium, aluminum, zirconium, hafnium, and titanium;
    element Y that is one or more selected from alkali metal elements; and
    silica;

    the catalyst having a peak height ratio $I_2/I_1$ of from 0 to 1.2, wherein $I_1$ represents the maximum peak height at $417 \pm 10$ cm$^{-1}$, and $I_2$ represents the maximum peak height at $1050 \pm 10$ cm$^{-1}$, as obtained by Raman spectroscopy.

2. The catalyst according to claim 1, wherein the $I_2/I_1$ is 0 to 0.5.

3. The catalyst according to claim 1 or 2, which is a catalyst for producing an unsaturated carboxylic acid and/or unsaturated carboxylic acid ester.

4. The catalyst according to any one of claims 1 to 3, wherein the element X comprises zirconium.

5. The catalyst according to any one of claims 1 to 4, wherein a content of the element X is from 0.3 to 10% by mass with respect to the total mass of the catalyst.

6. The catalyst according to any one of claims 1 to 5, wherein the element Y comprises cesium.

7. The catalyst according to any one of claims 1 to 6, wherein a content of the element Y is from 3 to 25% by mass with respect to the total mass of the catalyst.

8. The catalyst according to any one of claims 1 to 7, having a molar ratio MY/MX of from 1.3 to 8, wherein MX represents the number of moles of the element X, and MY represents the number of moles of the element Y.

9. The catalyst according to any one of claims 1 to 8, having a BET specific surface area of from 50 to 600 m$^2$/g.

10. A method of producing a catalyst, the method comprising steps of:

    (1) impregnating a silica-containing carrier with a solution or dispersion (liquid A) containing element X that is one or more selected from the group consisting of boron, magnesium, aluminum, zirconium, hafnium, and titanium, to obtain a solid component A;
    (2) subjecting the solid component A to heat treatment, to obtain a solid component B;
    (3) impregnating the solid component B with a solution or dispersion (liquid B) containing element Y that is one or more selected from alkali metal elements, to obtain a solid component C; and
    (4-1) subjecting the solid component C to heat treatment at a heat treatment temperature of 100 to 300°C, to obtain a catalyst;

    wherein, in the Step (2), the solid component A is subjected to heat treatment at a heat treatment temperature of from 150°C to 300°C for from 15 minutes to 24 hours.

11. The method of producing a catalyst according to claim 10, wherein, in the Step (2), the heating rate from the time when the solid component A reaches 120°C to the time when the solid component A reaches the heat treatment temperature is not more than 10°C/minute.

**12.** The method of producing a catalyst according to claim 10 or 11, wherein, in the Step (4-1), the solid component C is subjected to heat treatment for from 15 minutes to 24 hours.

**13.** A method of producing a catalyst, the method comprising steps of:

(1) impregnating a silica-containing carrier with a solution or dispersion (liquid A) containing element X that is one or more selected from the group consisting of boron, magnesium, aluminum, zirconium, hafnium, and titanium, to obtain a solid component A;
(2) subjecting the solid component A to heat treatment, to obtain a solid component B;
(3) impregnating the solid component B with a solution or dispersion (liquid B) containing element Y that is one or more selected from alkali metal elements, to obtain a solid component C; and
(4-2) subjecting the solid component C to heat treatment at a heat treatment temperature of from 400 to 450°C, to obtain a catalyst;

wherein, in the Step (4-2), the heating rate from the time when the solid component C reaches 120°C to the time when the solid component C reaches the heat treatment temperature is not more than 10°C/minute.

**14.** The method of producing a catalyst according to claim 13, wherein, in the Step (4-2), the solid component C is subjected to heat treatment for from 3 to 24 hours.

**15.** The method of producing a catalyst according to claim 13 or 14, wherein, in the Step (4-2), the heating rate from the time when the solid component C reaches 120°C to the time when the solid component C reaches the heat treatment temperature is from 2 to 8°C/minute.

**16.** A method of producing a catalyst, the method comprising steps of:

(1) impregnating a silica-containing carrier with a solution or dispersion (liquid A) containing element X that is one or more selected from the group consisting of boron, magnesium, aluminum, zirconium, hafnium, and titanium, to obtain a solid component A;
(2) subjecting the solid component A to heat treatment, to obtain a solid component B;
(3) impregnating the solid component B with a solution or dispersion (liquid B) containing element Y that is one or more selected from alkali metal elements, to obtain a solid component C; and
(4-3) subjecting the solid component C to heat treatment at a heat treatment temperature of more than 450°C and not more than 700°C, to obtain a catalyst; wherein, in the Step (4-3), the heating rate from the time when the solid component C reaches 120°C to the time when the solid component C reaches the heat treatment temperature is not more than 10°C/minute.

**17.** The method of producing a catalyst according to claim 16, wherein, in the Step (4-3), the solid component C is subjected to heat treatment for from 15 minutes to 24 hours.

**18.** The method of producing a catalyst according to claim 16 or 17, wherein, in the Step (4-3), the heating rate from the time when the solid component C reaches 120°C to the time when the solid component C reaches the heat treatment temperature is from 2 to 8°C/minute.

**19.** The method of producing a catalyst according to any one of claims 13 to 18, wherein, in the Step (2), the solid component A is subjected to heat treatment at a heat treatment temperature of from 100 to 200°C.

**20.** The method of producing a catalyst according to any one of claims 13 to 19, wherein, in the Step (2), the solid component A is subjected to heat treatment for from 15 minutes to 24 hours.

**21.** The method of producing a catalyst according to any one of claims 10 to 20, wherein the catalyst is a catalyst for producing an unsaturated carboxylic acid and/or unsaturated carboxylic acid ester.

**22.** The method of producing a catalyst according to any one of claims 10 to 21, wherein, in the Step (1), the element X comprises zirconium.

23. The method of producing a catalyst according to any one of claims 10 to 22, wherein, in the Step (3), the element Y comprises cesium.

24. A method of producing an unsaturated carboxylic acid and/or unsaturated carboxylic acid ester, the method comprising using the catalyst according to any one of claims 1 to 9, to produce an unsaturated carboxylic acid and/or unsaturated carboxylic acid ester from a carboxylic acid and/or carboxylic acid ester, and formaldehyde.

25. A method of producing an unsaturated carboxylic acid and/or unsaturated carboxylic acid ester, the method comprising using a catalyst produced by the method of producing a catalyst according to any one of claims 10 to 23, to produce an unsaturated carboxylic acid and/or unsaturated carboxylic acid ester from a carboxylic acid and/or carboxylic acid ester, and formaldehyde.

Fig. 1

## INTERNATIONAL SEARCH REPORT

| International application No. |
|---|
| **PCT/JP2022/022145** |

### A. CLASSIFICATION OF SUBJECT MATTER

***B01J 23/04***(2006.01)i; ***B01J 37/02***(2006.01)i; ***B01J 37/04***(2006.01)i; ***B01J 37/08***(2006.01)i; ***C07B 61/00***(2006.01)i;
***C07C 51/09***(2006.01)i; ***C07C 57/04***(2006.01)i; ***C07C 67/317***(2006.01)i; ***C07C 69/653***(2006.01)i
FI: B01J23/04 Z; B01J37/04 102; B01J37/08; B01J37/02 101D; C07C67/317; C07C69/653; C07C51/09; C07C57/04;
C07B61/00 300

According to International Patent Classification (IPC) or to both national classification and IPC

### B. FIELDS SEARCHED

Minimum documentation searched (classification system followed by classification symbols)

B01J21/00-38/74; C07C1/00-409/44;C07B61/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JST7580/ JSTChina (JDreamIII);CAplus (STN)

### C. DOCUMENTS CONSIDERED TO BE RELEVANT

| Category* | Citation of document, with indication, where appropriate, of the relevant passages | Relevant to claim No. |
|---|---|---|
| X | WO 2020/183193 A1 (LUCITE INTERNATIONAL UK LIMITED) 17 September 2020 (2020-09-17) claims, p. 1, lines 5-17, p. 28, line 25 to p. 33, line 6 | 1-9, 13-25 |
| X | WO 2020/184616 A1 (MITSUBISHI CHEMICAL CORPORATION) 17 September 2020 (2020-09-17) claims, paragraphs [0025], [0049], [0070], example 1 | 1-12, 21-25 |
| A | JP 2020-533170 A (LUCITE INTERNATIONAL UK LIMITED) 19 November 2020 (2020-11-19) examples 3, 20 | 1-25 |

☐ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **21 July 2022** | **02 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2022/022145**

| Patent document cited in search report | | | Publication date (day/month/year) | Patent family member(s) | | | Publication date (day/month/year) |
|---|---|---|---|---|---|---|---|
| WO | 2020/183193 | A1 | 17 September 2020 | CN | 113573807 | A | |
| | | | | KR | 10-2021-0134396 | A | |
| WO | 2020/184616 | A1 | 17 September 2020 | US | 2021/0387165 | A1 | |
| | | | | claims, paragraphs [0053]-[0056], [0103], [0142], example 1 | | | |
| | | | | CN | 113557086 | A | |
| | | | | KR | 10-2021-0133296 | A | |
| JP | 2020-533170 | A | 19 November 2020 | WO | 2019/053438 | A1 | |
| | | | | examples 3, 20 | | | |
| | | | | US | 2020/0269214 | A1 | |
| | | | | EP | 3681628 | A1 | |
| | | | | CN | 111344059 | A | |

Form PCT/ISA/210 (patent family annex) (January 2015)

23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 201953438 A

- WO 2020183193 A